# EUROPÄISCHE PATENTSCHRIFT

(11) **EP 0 969 833 B1**
(45) Veröffentlichungstag und Bekanntmachung des Hinweises auf die Patenterteilung: **28.05.2003**
(21) Anmeldenummer: 98913712.0
(22) Anmeldetag: 12.03.1998
(51) Int. Cl.: A61K 31/42, A61K 31/275, A61P 37/06

(54) **VERLÄNGERUNG DER EXPRESSION VON TRANSGENEN PROTEINEN DURCH IMMUNMODULIERENDE BEHANDLUNG MIT 15-DEOXYSPERGUALIN**
EXTENSION OF THE EXPRESSION OF TRANSGENIC PROTEINS BY IMMUNOMODULATION WITH 15-DEOXYSPERGUALINE WITH 15-DEOXYSPERGUALINE
PROLONGATION DE L'EXPRESSION DE PROTEINES TRANSGENIQUES PAR TRAITEMENT IMMUNOMODULANT AVEC DE LA 15-DEOXYSPERGUALINE

(30) Priorität: 21.03.1997 DE 19711800
(43) Veröffentlichungstag der Anmeldung: 12.01.2000
(73) Patentinhaber: HOECHST AKTIENGESELLSCHAFT, 65929 Frankfurt am Main (DE)
(72) Erfinder: SEEMANN, Gerhard, D-35041 Marburg-Elnhausen (DE); CICHON, Günter, D-10785 Berlin (DE)
(86) Internationale Anmeldenummer: EP9801438
(87) Internationale Veröffentlichungsnummer: WO98042338

(56) Entgegenhaltungen:
- EP-A- 0 551 230
- EP-A- 0 607 775
- EP-A- 0 821 952
- WO-A-96/40170
- HAYASHI ET AL.: "Adenovirus-Mediated Gene Transfer To The Xenogeneid Liver in Liver Xenotransplantation: The Transduction of Complement Regulatory Factor Genes (DAF and HRF20)" CELL TRANSPLANTATION, Bd. 5, Nr. 5 suppl 2, 1996, Seite 73 XP002074074
- GREENE ET AL.: "INHIBITION OF DIHYDROOROTATE DEHYDROGENASE BY THE IMMUNOSUPPRESSIVE AGENT LEFLUNOMIDE" BIOCHEM. PHARMACOL., Bd. 50, Nr. 6, 1995, Seiten 861-867, XP002074075
- BARTLETT ET AL.: "Leflunomide (HWA 486), a novel immunomodulating compound for the treatment of autoimmune disorders and reactions leading to transplantation rejection" AGENTS ACTIONS, Bd. 32, Nr. 1-2, 1991, Seiten 10-21, XP002074076
- SOLBACH ET AL.: "PROTECTIVE EFFECT OF LEFLUNOMIDE ON THE NATURAL COURSE OF LEISHMANIA MAJOR-INDUCED DISEASE IN GENETICALLY SUSCEPTIBLE BALB/c MICE" INT. J. IMMUNOPHARMACOL., Bd. 17, Nr. 6, 1995, XP002074077
- KALDEN ET AL.: "New therapeutic approaches in autoimmune rheumatic diseases, with special emphasis on rheumatoid arthritis" BRITISH J. RHEUMATOL., Bd. 34, Nr. 3, 1995, Seiten 193-196, XP002074078
- ZIELINSKI T ET AL: "LEFLUNOMIDE, A REVERSIBLE INHIBITOR OF PYRIMIDINE BIOSYNTHESIS?" INFLAMMATION RESEARCH, Bd. 44, Nr. 2, August 1995, Seite S207/S208 XP002061643
- REYNOLDS: "MARTINDALE - THE EXTRA PHARMACOPOEIA. " 1996 , ROYAL PHARMACEUTICAL SOCIETY , LONDON XP002074427 224540 siehe Seite 557-562 - Seite 599-600
- BURG G.: "Dermatologie 1997: Immunintervention auf dem Vormarsch" SCHWEIZERISCHE MEDIZINISCHE WOCHENSCHRIFT, Bd. 128, Nr. 1-2, 1998, Seiten 18-20, XP002074079

## Beschreibung

Die Erfindung betrifft die Verwendung von 15-Deoxyspergualin zur Herstellung eines Arzneimittels, um die Toleranz eines Säugetiers gegenüber transgenen Zellen zu erhöhen, sowie ein Verfahren zum Identifizieren hierfür geeigneter Immunsuppressiva. Durch den Einsatz derartiger Arzneimittel wird die Produktion des transgenen Expressionsproduktes auch nach Absetzen der immunsuppressiven Behandlung deutlich verlängert.

Es ist bekannt, daß die Einschleusung von genetischem Material in Zellen eines Säugetiers, eines Menschen oder auch diverser Tiere wie Pferde, Schafe, Kühe, Ziegen, Schweine, Hunde, Mäuse oder Ratten, in vivo häufig eine immunologische Reaktion hervorruft. So werden die transgenen Zellen beispielsweise durch zytotoxische Immunzellen abgetötet und damit durch diese zelluläre Immunreaktion die über das eingeschleuste genetische Material bewirkte Expression eines oder mehrerer Proteine oder Peptide beendet. Eine humorale Immunreaktion wiederum erschwert oder verhindert die erneute Einschleusung des genetischen Materials, z.B. durch die Neutralisation der Vektoren mittels spezifischer Antikörper (Tripathy S.K. et al., nat. Med:2(5), 1996, Seiten 545-550; Jang J. et al., Gene Ther. 3(2): 1996, Seiten 137-144).

Um eine unerwünschte Immunreaktion bei einer transgene Zellen erzeugenden Gentherapie zu unterdrücken oder zu verhindern, wurden daher immunsuppressiv wirkende Substanzen im Rahmen einer Begleittherapie eingesetzt. Als nachteilig erwiesen sich jedoch die zum Teil damit verbundenen unangenehmen Nebenwirkungen sowie die erhöhte Infektanfälligkeit von immunsupprimierten Organismen, die einer längeren Gabe von Immunsuppressiva entgegenstehen (Lochmüller et al. Gene Ther. 3(8), 1996, Seiten 706-716).

In WO 96/12406 wurde beschrieben, daß durch eine immunsuppressive Begleittherapie die humorale Immunantwort gegen einen Adenovirus-Vektor unterdrückt werden kann und daß dadurch eine wiederholte Vektorgabe ermöglicht wird. Neben der Verwendung von Steroiden wie Dexamethason werden vor allem Cyclosporin A und 1-Amino-19-guanidin-11-hydroxy-4,9,12-triazanondecan-10,13-dion-trihydrochlorid (Deoxyspergualin; im folgenden DSG genannt) für diesen Zweck empfohlen.

Aufgabe der Erfindung ist die Identifizierung von Substanzen, die nachhaltig - also auch nach dem Absetzen der immunsuppressiven Begleittherapie - das rasche Zerstören der transgenen Zellen verhindern und somit die Toleranz eines Säugetiers gegenüber transgenen Zellen erhöhen. Damit bleibt die Expression des oder der transgenen Produkte in vivo länger erhalten. Folglich könnte eine wiederholte Applikation des genetischen Materials unterbleiben oder zumindest von der Häufigkeit her eingeschränkt werden.

Es wurde gefunden, daß 15-Deoxyspergualin die gewünschte Wirksamkeit aufweist. Nicht geeignet waren beispielsweise FK 506 und das in WO 96/12406 besonders empfohlene Cyclosporin A.

Von der Erfindung umfaßt wird die Verwendung von 15-Deoxyspergualin zur Herstellung eines Arzneimittels zur Erhöhung der Toleranz eines Säugetiers, insbesondere des Menschen, gegenüber transgenen Zellen.

Immunsuppressiva sind Substanzen, die in der Lage sind, immunologische Reaktionen zu unterdrücken oder abzuschwächen. Sie werden vor allem in der Transplantationsmedizin und zur Behandlung von Autoimmunkrankheiten verwendet. Geeignete Immunsuppressiva sind beispielsweise N-(4-Trifluormethylphenyl-5-methyl-isoxazol-4-carboxamid (Leflunomid), DSG, Anti-T-Zell-Antikörper, Corticosteoride, Azathioprin, Cyclophosphamid oder Methotrexat. Die chemische Struktur des Immunsuppressivums ist - vom allgemeinen Erfindungsgedanken gesehen - unerheblich, da das entscheidende Merkmal in seiner Wirkung begründet ist, die Toleranz gegenüber transgenen Zellen zu erhöhen.

Als Toleranz gegenüber transgenen Zellen wird verstanden, daß die transgenen Zellen nach Beendigung der erfindungsgemäßen immunsuppressiven Begleittherapie länger in der Lage sind, die transgenen Expressionsprodukte in vivo herzustellen als sie es in Säugetieren einer nicht immunsuppremierten Kontrollgruppe vermögen.

Transgene Zellen sind Zellen jeglicher Art, also tierische, pflanzliche oder bakterielle Zellen, bevorzugt Zellen von Säugetieren, ganz besonders bevorzugt menschliche Zellen, in die genetisches Material eingeschleust worden ist.

Unerheblich ist für den allgemeinen Erfindungsgedanken das mit der Herstellung der transgenen Zellen verbundene Ziel, beispielsweise eine gentherapeutische Behandlung oder aber die Nutzung eines Tieres mit transgenen Zellen als Wirkstoffproduzent. Des weiteren ist auch weder das Herstellverfahren der transgenen Zellen noch das genetische Material per se für die Erfindung von ausschlaggebender Bedeutung. Das eingeschleuste genetische Material kann beispielsweise aus einer oder mehreren Desoxyribonukleinsäure- und/oder Ribonukleinsäureketten bestehen. Die Nukleinsäureketten können Promotoren, diverse andere genregulatorische Steuerungsfunktionen, Sequenzen für einen spezifischen Einbau ins zelluläre Genom und/oder Gensequenzen enthalten, die für spezifische Proteine oder Peptide kodieren. Das eingeschleuste genetische Material kann gegenüber dem zellulären Genom artfremd oder artgleich sein oder sogar vom selben Individuum abstammen. Auch eine Kombination derselben ist möglich. Das oder die spezifischen transgenen Expressionsprodukte können nicht-natürliche oder natürliche, beispielsweise menschliche, tierische, pflanzliche, bakterielle oder virale Proteine oder Peptide darstellen. Beispiele sind Enzyme, Rezeptoren, Botenstoffe, Hormone, Wachstumsfaktoren, Gerinnungsfaktoren, Apolipoproteine, den Stoffwechsel oder die Zellteilung beeinflußende Faktoren, entzündungshemmende Faktoren, Inhibitoren oder Aktivatoren des Zellzyklusses sowie intrazellulärer Signalketten, Tumorsuppressoren, Elemente des Zytoskeletts oder des Bindegewebes, Antigene von Krankheitserregern oder Parasiten sowie tumorassoziierte Antigene. Weitere Beispiele sind Insulin; Hirudin; Faktor VIII; Faktor IX; Faktor XIII; von Willebrand-Faktor; Antikörper; Erythropoitin; menschliches Wachstumshormon; "Growth factors" wie EGF, TGFα, TGFβ, GM-CSF, PDGF, Nerve growth factor und andere; "Tumor necrosis factor"; Interferone; Interleukine; p53; Tumortherapie; Hepatitis-Virus-Antigene; HIV-Antigene, Herpes-Virus-Antigene; Borrelia-Antigene; Plasmodium-Antigene; Trypanosomen-Antigene, Taenia-Antigene oder Humane-β-glucuronidase.

Mit der erfindungsgemäßen Verwendung des oder der Immunsuppressiva wird insbesondere eine Inhibition der zellulären Immunantwort gegen die transgenen Zellen erreicht. Die Erfindung ist ferner gekennzeichnet, daß etwa 15 Tage nach Absetzen des immunsuppressiv wirkenden Arzneimittels oder der Arzneimittelkombination in den behandelten Säugetieren noch mehr als das 1,5-fache, bevorzugt mehr als doppelte, ganz besonders bevorzugt mehr als das 5fache an transgenem Expressionsprodukt erzeugt werden kann wie in Säugetieren einer nicht immunsuppremierten Kontrollgruppe. Der Nachweis des exprimierten transgenen Produktes muß nicht am 15. Tag erfolgen; es ist auch möglich Messungen vor oder nach diesem Zeitpunkt durchzuführen. Entscheidend ist, daß nicht schon wenige Tage, beispielsweise 10 oder 20 Tage, nach Absetzen der immunsuppressiven Behandlung, das transgene Expressionsprodukt nur in gleicher oder geringerer Menge hergestellt wird wie in nicht-immunsuppremierten Organismen einer Kontrollgruppe.

Die erfindungsgemäße Verwendung umfaßt des weiteren eine Verabreichung des Arzneimittels oder der Arzneimittelkombination vor, während und/oder nach der Applikation der in vitro erzeugten transgenen Zellen oder deren Erzeugung in vivo. Ebenso umfaßt wird die erfindungsgemäße Verwendung zur Unterstützung einer transgene Zellen erzeugenden gentherapeutischen Behandlung.

Bei der zu unterstützenden gentherapeutischen Behandlung wird in vitro oder in vivo genetisches Material in Form von einer oder mehreren Nukleinsäureketten in Zellen eingeschleust. Dies erfolgt beispielsweise mit Hilfe viraler Vektoren, wie Adenoviren, Retroviren oder Herpesviren, oder anderen Methoden, beispielsweise über Transfektion, durch direkte Injektion, per "Gene gun", oder mit Hilfe von Liposomen, Virosomen oder Rezeptor-vermittelten Transportsystemen.

Mit der zu unterstützenden Gentherapie können Erkrankungen behandelt werden, bei denen ein Protein oder Peptid nicht, unzureichend oder nur fehlerhaft im Körper des Säugetiers hergestellt wird, aber sie findet auch Anwendung, um einen Impfschutz gegen diverse Krankheitserreger oder gegen entartete körpereigene Zellen zu erzeugen sowie für die Herstellung nicht-natürlicher Proteine oder Peptide in Körperzellen, die Aktivitäten von Enzym- oder Signalkaskaden fördernd oder inhibierend beeinflussen oder für die Herstellung von Enzymen, die spezifische Prodrugs aktivieren. So kann die zu unterstützende Gentherapie eingesetzt werden zur Behandlung von angeborenen Erkrankungen wie der zystischen Fibrose, der Hämophilie, der familiären Hypercholesterinämie, der Sichelzellenanämie, der Phenylketonurie; von Stoffwechselstörungen, wie Diabetes; von Entzündungen; von Nerven- und Himerkrankungen wie Parkinson, Alzheimer oder Jakop-Kreuzfeld-Syndrom; von rheumatischen Erkrankungen, Osteoathritis, Osteopörose oder Arthrose, von Krebserkrankungen; von Infektionserkrankungen wie z.B. AIDS oder Hepatitis oder von Hormon- und Wachstumsstörungen. Die Erzeugung eines Impfschutzes gegen Krankheitserreger wie Viren, Bakterien, Pilze, ein- und mehrzellige Parasiten sowie gegen entartete körpereigene Zellen, beispielsweise Tumorzellen ist ein weiterer zu unterstützender gentherapeutischer Einsatzbereich.

Das erfindungsgemäße Arzneimittel kann beispielsweise oral, intravenös, subkutan, intraperitoneal, perkutan, kutan, topisch, inhalativ, intramuskulär, intrathekal, intraokulär, okulär, buccal, nasal oder rektal, bevorzugt intravenös oder oral verabreicht werden.

Die folgenden Beispiele dienen der Erläuterung der Erfindung. Sie sind nicht als Einschränkung irgendeiner Art zu verstehen.

### Beispiel 1:

Cyclosporin A (50 - 100 mg/kg/Tag) und DSG (10 mg/kg/Tag) wurde den Mäusen über einen Zeitraum von 21 Tagen nach Gabe eines rekombinanten Adenovirus (Reportergen β-Galactosidase; Bett et al. Proc. Natl. Acad. Sci. USA, 91 (1994), Seiten 8802-8806) verabreicht.

Die Kontrollgruppe erreicht ein Maximum der Reportergenexpression in der Leber am 6. Tag, danach wird die Expression durch die Aktivität zytotoxischer T-Zellen kontinuierlich abgebaut und erreicht nach etwa 21 Tagen Ausgangsniveau.

Sowohl in der Cyclosporin A-, als auch in der DSG-Gruppe hält die Expression über den Behandlungszeitraum von 21 Tagen unvermindert an.

In der Kontrollgruppe konnte bereits 5 Tage nach Vektorgabe die Bildung von Antikörpern nachgewiesen werden. In der Cyclosporin A- und der DSG-Gruppe wurde Antikörperproduktion in den oben angegebenen Dosen vollständig aufgehoben.

Nach Absetzen der Immunsupressiva fiel die Reportergenexpression in der Cyclosporin A-Gruppe über einen Zeitraum von 21 Tagen (42 Tage nach Vektorgabe) auf Ausgangsniveau während in der DSG-Gruppe noch eine massive Genexpression nachweisbar war.

In etwa 50 % der Tiere die nur für 5 Tage nach Vektorgabe mit DSG behandelt wurden konnte am 42. Tag nach Vektorgabe noch etwa 10 % der Maximalexpression gemessen werden. Offensichtlich hemmt DSG besonders die Frühphase der T-Zellstimulation sehr effektiv.

### Beispiel 2

Am Tag 0 wurde allen Mäusen eine Dosis von 1x10¹⁰ Adenoviren über die Schwanzvene appliziert. Die rekombinanten Adenoviren tragen als serumständiges Reportergen das Gen für humanes alpha 1-Antitrypsin. Dieses Gen hat sich als ein praktikabler Reporter erwiesen, da es in der Maus nicht antigen wirkt, aber dennoch mit einem spezifischen Antikörper vom murinen alpha 1-Antitrypsin unterschieden werden kann. Die Halbwertzeit der Antiprotease ist etwa 3 - 4 Tage, so daß der Serumspiegel recht gut die aktuelle Syntheseleistung der Leber spiegelt.

Das Gesamtkollektiv wurde in fünf Gruppen mit jeweils 4 Tieren unterteilt, die wie folgt behandelt wurden:
1. Gruppe: Kontrollgruppe - keine immunsupressive Behandlung, Applikation von Kochsalzlösung i.p. für die Dauer von 5 Tagen nach Virusgabe.
2. Gruppe: FK 506 Gruppe - Applikation von 1 mg/kg/Tag FK 506 i.p. beginnend 1 Tag vor Virusgabe und danach für eine Dauer von 5 Tagen.
3. Gruppe: Cyclosporin A -Gruppe - Applikation von 20 mg/kg/Tag i.p. beginnend 1 Tag vor Virusgabe und danach für eine Dauer von 5 Tagen.
4. Gruppe: DSG-Gruppe -Applikation von 10 mg/kg/Tag i.p. beginnend 1 Tag vor Virusgabe und danach für die Dauer von 5 Tagen.
5. Gruppe: DSG ++ - Gruppe - Applikation von 10 mg/kg/Tag i.p. beginnend 1 Tag vor Virusgabe und danach für die Dauer von fünf Tagen täglich und anschließend zweimal wöchentlich für die Dauer von 150 Tagen.

NMRI-Mäuse wurden verwendet, da diese Mäuse durch ihre genetische Heterogenität ein immunologisches Reaktionsmuster entwickeln, das dem einer humanen Population näher kommt als reine Inzuchtstämme, der Preis ist eine erhöhte Variabilität in der Immunologischen Reaktionsfähigkeit. Trotzdem bilden die einzelnen Gruppen signifikante Reaktionsmuster aus.

Tabelle 1 zeigt die Mittelwerte des Serumspiegels von humanen α₁-Antitrypsin.

**Tabelle 1**

| Tage | FK 506 | Cyclosporin A | Kontrolle | DSG | DSG++ |
|---|---|---|---|---|---|
| 5 | 1725000 | 1518750 | 1453750 | 2775000 | 2775000 |
| 10 | 1278750 | 660000 | 616250 | 1560000 | 677500 |
| 20 | 376250 | 264875 | 1055000 | 1826250 | 1178750 |
| 30 | 17475 | 77787 | 117812 | 1001250 | 1082500 |
| 60 | 4258 | 23875 | 37625 | 213500 | 297500 |
| 100 | 313 | 1876 | 12475 | 51750 | 68750 |
| 150 | 1,5 | 8 | 2425 | 13125 | 17125 |
| 200 | 1,5 | 1,5 | 1797 | 10625 | 14350 |

Bereits eine 5-tägige Behandlung mit DSG führt zu einer deutlichen Erhöhung und Verlängerung der Antitrypsinexpression. Der Mengenfaktor beträgt 15 Tage nach Absetzen von DSG etwa 1,8. Die kontinuierliche Behandlung (2 x wöchentlich) vermag diesen Effekt nicht mehr dramatisch zu steigern. Durch die kontinuierliche DSG-Behandlung wird die Expression nur geringfügig über die transiente Gabe gesteigert, aber der Antikörperload gegen virale Proteine wird etwa um 90 % nach 150 Tagen reduziert (siehe Tabelle 2). Bereits die transiente Gabe von DSG reduziert im Vergleich zur Kontrolle die Antikörperantwort um mehr als 60 % bei gleichzeitig deutlich positivem Effekt auf die Expression.

**Tabelle 2:**

| Mittlerer Antikörper-Titer | | | | | |
|---|---|---|---|---|---|
| Tage | Kontrolle | DSG | DSG++ | FK 506 | Cyclosporin A |
| 30 | 54188 | 26310 | 13005 | 65536 | 47923 |
| 100 | 59707 | 31029 | 11251 | 43832 | 32994 |
| 150 | 68555 | 25149 | 7335 | 25149 | 28588 |
| 200 | 45641 | 20586 | 9669 | 17710 | 26021 |

Interessant ist, daß die kurzfristige Gabe von FK 506 und Cyclosporin A kontraproduktiv im Sinne einer Langzeitexpression ist. Möglicherweise reagiert das Immunsystem mit einer überschießenden Aktivität nach Absetzen der Immunsupressiva und eliminiert die transgenen Hepatozyten schneller als in der Kontrollgruppe.

## Patentansprüche

1. Verwendung von 15-Deoxyspergualin zur Herstellung eines Arzneimittels zur Erhöhung der Toleranz eines Säugetiers, insbesondere des Menschen, gegenüber transgenen Zellen, nach Absetzen der immunosuppressiven Begleittherapie, wobei die transgenen Zellen mittels eines rekombinanten Adenovirus-Vektors transfiziert sind.

2. Verwendung nach Anspruch 1, **dadurch gekennzeichnet, daß** das Arzneimittel vor, während und/oder nach der Applikation der in vitro erzeugten transgenen Zellen oder der in vivo-Erzeugung der transgenen Zellen zu verabreichen ist.

3. Verwendung nach Anspruch 1 oder 2, wobei die transgenen Zellen im Rahmen einer gentherapeutischen Behandlung erzeugt wurden.

4. Verwendung nach Anspruch 3, **dadurch gekennzeichnet, daß** die gentherapeutische Behandlung einzusetzen ist zur Behandlung aller Erkrankungen, bei denen ein Protein oder Peptid nicht, unzureichend oder nur fehlerhaft im Körper des Säugetiers, insbesondere des Menschen, hergestellt wird.

5. Verwendung nach Anspruch 3, **dadurch gekennzeichnet, daß** die gentherapeutische Behandlung einzusetzen ist zur Behandlung von angeborenen Erkrankungen wie der zystischen Fibrose, der familiären Hypercholesterinämie, der Hämophilie, der Sichelzellenanämie, von Nervenund Hirnerkrankungen wie Parkinson-, Alzheimer- oder Jakop-Kreuzfeld-Syndrom; von rheumatischen Erkrankungen, Osteoarthritis, Osteopörose oder Arthrose, der Phenylketonurie; von Stoffwechselstörungen, wie Diabetes; von Entzündungen; von Krebserkrankungen; von Infektionserkrankungen, beispielsweise AIDS oder Hepatitis oder von Hormon- und Wachstumsstörungen.

6. Verwendung von Anspruch 3, **dadurch gekennzeichnet, daß** die gentherapeutische Behandlung einzusetzen ist>, um einen Impfschutz zu erzeugen gegen Krankheitserreger wie Viren, Bakterien, Pilze, ein- und mehrzellige Parasiten sowie gegen entartete körpereigene Zellen wie Tumorzellen.

7. Verwendung nach mindestens einem der Ansprüche 1 bis 6, **dadurch gekennzeichnet, daß** das Arzneimittel oral, intravenös, subkutan, intraperitoneal, perkutan, kutan, topisch, inhalativ, intramuskulär, intrathekal, intraokulär, okulär, buccal, nasal oder rektal, bevorzugt intravenös oder oral zu verabreichen ist.

## Claims

1. The use of 15-deoxyspergualin for the production of a pharmaceutical for increasing the tolerance of a mammal, in particular man, to transgenic cells, after discontinuing the immunosuppressant concomitant therapy, the transgenic cells being transfected by means of a recombinant adenovirus vector.

2. The use as claimed in claim 1, wherein the pharmaceutical is to be administered before, during and/or after the administration of the transgenic cells produced in vitro or of the in-vivo production of the transgenic cells.

3. The use as claimed in claim 1 or 2, wherein the transgenic cells were produced in the course of a gene therapy treatment.

4. The use as claimed in claim 3, wherein the gene therapy treatment is to be employed for the treatment of all disorders in which a protein or peptide is not produced, is produced inadequately or only produced defectively in the body of the mammal, in particular of man.

5. The use as claimed in claim 3, wherein the gene therapy treatment is to be employed for the treatment of hereditary disorders such as cystic fibrosis, familial hypercholesterolemia, hemophilia, sickle cell anemia; of nerve and brain disorders such as Parkinson's, Alzheimer's or Creutzfeld-Jakob syndrome; of rheumatic disorders, osteoarthritis, osteoporosis or arthrosis, of phenylketonuria; of metabolic disorders, such as diabetes; of inflammations; of carcinomatous disorders; of infectious disorders, for example AIDS or hepatitis or of hormone and growth disorders.

6. The use as claimed in claim 3, wherein the gene therapy treatment is to be employed in order to generate a vaccine protection against disease pathogens such as viruses, bacteria, fungi, mono-and multicellular parasites and also against abnormal body cells such as tumor cells.

7. The use as claimed in at least one of claims 1 to 6, wherein the pharmaceutical is to be administered orally, intravenously, subcutaneously, intraperitoneally, percutaneously, cutaneously, topically, by inhalation, intramuscularly, intrathecally, intraocularly, ocularly, buccally, nasally or rectally, preferably intravenously or orally.

## Revendications

1. Utilisation de la 15-désoxyspergualine pour la préparation d'un médicament pour l'augmentation de la tolérance d'un animal mammifère, en particulier de l'homme, vis-à-vis des cellules transgéniques, après l'arrêt de la thérapie d'accompagnement immunodépressive, les cellules transgéniques étant transfectées par un vecteur adénovirus recombinant.

2. Utilisation selon la revendication 1, **caractérisée en ce que** le médicament doit être administré avant, au cours et/ou après l'application des cellules transgéniques produites *in vitro* ou au cours de la production *in vivo* des cellules transgéniques.

3. Utilisation selon la revendication 1 ou 2, où les cellules transgéniques éta,t produites dans le cadre d'un traitement thérapeutique.

4. Utilisation selon la revendication 3, **caractérisée en ce que** le traitement de thérapie génique doit être appliqué pour le traitement de toutes les maladies dans lesquelles une protéine ou un peptide n'est pas produits, sont produits d'une manière insuffisante ou seulement d'une manière défectueuse dans l'organisme de l'animal mammifère, en particulier de l'homme.

5. Utilisation selon la revendication 3, **caractérisée en ce que** le traitement par thérapie génique doit être appliqué pour le traitement de maladies congénitales telles que la mucoviscidose, l'hypercholestérolémie, l'hémophilie, la sicklémie, des maladies neurologiques et cérébrales comme la maladie de Parkinson, d'Alzheimer ou le syndrome de Creutzfeldt-Jakob ; de maladie rheumatiques, d'ostéoarthrose, d'ostéoporose ou d'arthrose, de phénylcétonurie ; de troubles de métabolisme, comme le diabète ; de maladies infectieuses, par exemple le SIDA ou l'hépatite ou de troubles hormonales ou de croissance.

6. Utilisation selon la revendication 3, **caractérisée en ce qu'**on doit appliquer le traitement thérapeutique pour produire une protection assurée par un vaccin contre des agents pathogènes comme les virus, les bactéries, les champignons, les parasites uni- et pluricellulaire ainsi que contre des cellules dégénérées endogènes, comme les cellules tumorales.

7. Utilisation selon l'une des revendications 1 à 6, **caractérisée en ce que** le médicament doit être administré par voies orale, intraveineuse, sous-cutanée, intrapéritonéale, percutanée, cutanée, topique, par inhalation, intramusculaire, intrathécale, intraoculaire, oculaire, buccale, nasale ou rectale, de préférence intraveineuse ou orale.
